# EUROPEAN PATENT SPECIFICATION

(11) **EP 0 594 225 B1**
(45) Date of publication and mention of the grant of the patent: **17.09.1997**
(21) Application number: 93120996.9
(22) Date of filing: 23.01.1990
(51) Int. Cl.: C07C 13/48, C07C 2/72

(54) **Process for preparing polyalkyl tetrahydronaphtalenes**
Verfahren zur Herstellung von Polyalkyltetranaphtalenen
Procédé pour la préparation de polyalkyltetrahydronaphtalènes

(30) Priority: 27.01.1989 US 303422; 27.01.1989 US 303419; 27.01.1989 US 303365; 27.01.1989 US 303366
(43) Date of publication of application: 27.04.1994
(62) Divisional of application: 90300678.1
(73) Proprietor: Union Camp Corporation, Wayne New Jersey 07470 - 2066 (US)
(72) Inventor: Frank, Walter C., Holland, PA 18966 (US)
(74) Representative: Jones, Helen Marjorie Meredith

(56) References cited:
- US-A- 4 551 373
- US-A- 4 877 911
- CHEMICAL ABSTRACTS, vol. 97, no. 5, 2 August 1982, Columbus, Ohio, US; abstract no. 38653f, page 545 ; & JP-A-57 040 420 (NITTO RIKEN INDUSTRIES) 06 March 1982
- CHEMICAL ABSTRACTS, vol. 95, no. 17, 26 October 1981, Columbus, Ohio, US; abstract no. 150266s, page 630 ; & JP-A-56 039 026 (TAKASAGO PERFUMERY CO) 14 April 1981

## Description

The present invention relates to an improved process for the production of polyalkyl tetrahydronaphthalenes, particularly 1,1,3,4,4,6-hexamethyl-1,2,3,4-tetrahydronaphthalene, the latter compound referred to herein as "HMT".

HMT and other alkyl-substituted tetrahydronaphthalenes are of significant importance to the perfumery as well as other industries. By conventional acylation processes, HMT, for example, can be converted to 7-acetyl-1,1,3,4,4,6-hexamethyl-1,2,3,4-tetrahydronaphthalene, a well known musk perfume. Because of their clean musk fragrance and their ability to retain that fragrance over long periods of time, these HMT derivatives are of great commercial value as synthetic musk perfume substitutes for the expensive, natural musk perfumes of the macrocyclic ketone series. Consequently, various synthetic methods have been proposed for the production of HMT, as well as other related intermediates of HMT useful in the perfumery or other industries.

For example, Cobb, U.S. Patent No. 4,551,573, discloses a process for the alkylation of aromatic compounds with olefinic compounds in the presence of a catalyst consisting essentially of aluminum halide and elemental iodine. Examples of aromatic compounds described as suitable for use in the process include para-cymene, and olefinic compounds discussed include 2,3-dimethyl-2-butene, isobutylene and neohexene (3,3-dimethyl-1-butene). A mixture of olefinic compounds can also be employed, in which case it is noted that one of the olefins may function as a sacrificial agent. The products of the alkylation reaction described include indanes and tetralins.

Wood et al., U.S. Patent No. 3,856,875 discusses a process for the preparation of HMT wherein an equivalent or excess amount of para-cymene is reacted with a substantially equal molar solution of neohexene and a tertiary alkyl halide in the presence of an effective amount of an anhydrous aluminum halide catalyst suspended in a reaction-compatible solvent. Although any tertiary alkyl halide can be employed in the disclosed process, tertiary butyl chloride, tertiary amyl chloride and 2,5-dichloro-2,5-dimethylhexane are noted as preferred. The process is described as having a solvent dependency, with the satisfactory solvents being ethylene dichloride, chloroform, methylene dichloride, 1,1,2,2-tetrachloroethane, 1,2-dichloroethylene, 1,2,3-trichloropropane, 1,1,2-trichloroethane, monochlorobenzene, fluorobenzene, ortho-dichlorobenzene, and para-xylene. Numerous solvents were stated to be unsatisfactory for use in the disclosed process, such solvents including nitromethane, benzene, nitrobenzene, para-cymene, n-hexane, 1,2,2-trichloroethylene, carbon tetrachloride, 1,1,1-trichloroethane, carbon disulfide, 1,1,2,2,2-pentachloroethane, 1,2-dichloropropane, 1,1-dichloroethylene, and 1,1-dichloroethane. These unsatisfactory solvents are said to yield substantially poorer results.

Wood, U.S. Patent No. 3,246,044, discloses a process for preparing HMT which includes reacting an alpha,para-dimethylstyrene derivative such as dimethyl-para-tolyl-carbinyl halide, and neohexene in the presence of a catalyst such as aluminum chloride, aluminum bromide and ferric chloride, or other Friedel-Crafts catalysts, at low temperatures. Suitable solvents are listed as ethylene dichloride or carbon tetrachloride, or other inert chlorinated hydrocarbon solvents. It is noted that other solvents such as nitrobenzene and nitromethane, may be used, but the yield of desired product is indicated as generally being lower when such solvents are employed.

Sato et al., U.S. Patent No. 4,284,818, describes a process for producing HMT comprising reacting para-cymene with a 2,3-dimethyl butene using a catalytic amount of anhydrous aluminum halide in the presence of a secondary alkyl halide, tertiary alkyl halide, propargyl halide or allyl halide. It is noted that both the 2,3-dimethyl-1-butene and 2,3-dimethyl-2-butene can be employed as the 2,3-dimethyl butene reagent, however, 2,3-dimethyl-1-butene was said to yield better results. The reaction is generally carried out using a solvent, such solvents including aliphatic hydrocarbons, halogenated aromatic hydrocarbons, and halogenated aliphatic hydrocarbons.

Japanese Patent Publication SHO 57-40420 discusses a method of making HMT characterized by reacting para-cymene and neohexene in the presence of anhydrous aluminum halide as catalyst. Suitable anhydrous aluminum halides are said to include aluminum chloride. The reaction is generally carried in a solvent, however, it is noted that it is possible to conduct the reaction without any additional solvent using excess para-cymene. Examples of suitable solvents are methylene chloride, ethylene chloride, chloroform and other inactive fatty hydrocarbon halides. Other solvents such as aromatic hydrocarbon halides, fatty hydrocarbons, aromatic hydrocarbons, etc., can be used, but it is noted that the use of such solvents generally lowers the yield of the desired end product.

Kahn, U.S. Patent No. 3,379,785, relates to a process for preparing polyalkyl tetrahydronaphthalenes, and more specifically, a process for preparing HMT. The process involves the reaction of a substituted styrene and a 2,3-dimethylbutene, said reaction being carried out at elevated temperatures and in the presence of a cation exchange resin. The 2,3-dimethylbutene reactant employed is disclosed as comprising either 2,3-dimethyl-1-butene, 2,3-dimethyl-2-butene, or mixtures thereof. The preferably employed solvent comprises an aromatic hydrocarbon, such as, for example, benzene, toluene, ethylbenzene, or a xylene.

Suzukamo et al., U.S. Patent No. 4,767,882, discloses a process for preparing a tetrahydronaphthalene derivative in an optically active state which comprises reacting a benzene compound and a pyrocine compound in the presence of a Lewis acid, or, alternatively, reacting the benzene with the pyrocine compound in the presence of an acid catalyst followed by treatment of the resultant product with the Lewis acid.

These prior art processes suffer from various drawbacks, including low conversion of reactants, poor selectivity to the desired products, sluggish reaction rates, unacceptably low temperature requirements, unsafe solvent systems, or oxygen sensitivity. New and/or better processes are needed. The present invention is directed to this important end.

### SUMMARY OF THE INVENTION

The present invention provides a process for the production of polyalkyl tetrahydronaphthalenes wherein a cyclialkylation reaction between an olefinic compound of the general Formula 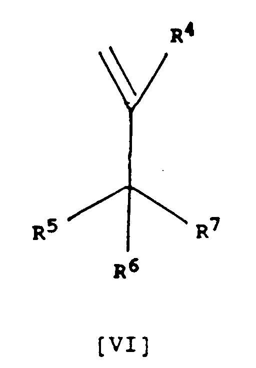 and a substituted benzene compound is carried out in the presence of a hydride abstracting reagent, an alkyl halide, a Lewis acid, and a phase transfer agent. In some embodiments, the foregoing process is specifically carried out in the absence of elemental iodine.

The present invention provides a further process for the production of polyalkyl tetrahydronaphthalenes wherein a cyclialkylation reaction between an olefinic compound of the general Formula 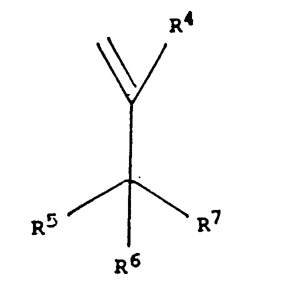 and a substituted benzene compound is carried out in the presence of an alkyl halide, a Lewis acid and a phase transfer agent.

The subject processes, which in some embodiments may also be practiced in an unhalogentated hydrocarbon solvent, produce the desired compounds in a surprisingly high yield, with a surprisingly high selectivity to the desired product, and at a relatively high rate of reaction, using better, more convenient or less expensive process methodology than many processes known heretofore.

Specifically, the present invention pertains to a process for producing polyalkyl tetrahydronaphthalenes, such as those represented by the Formulas 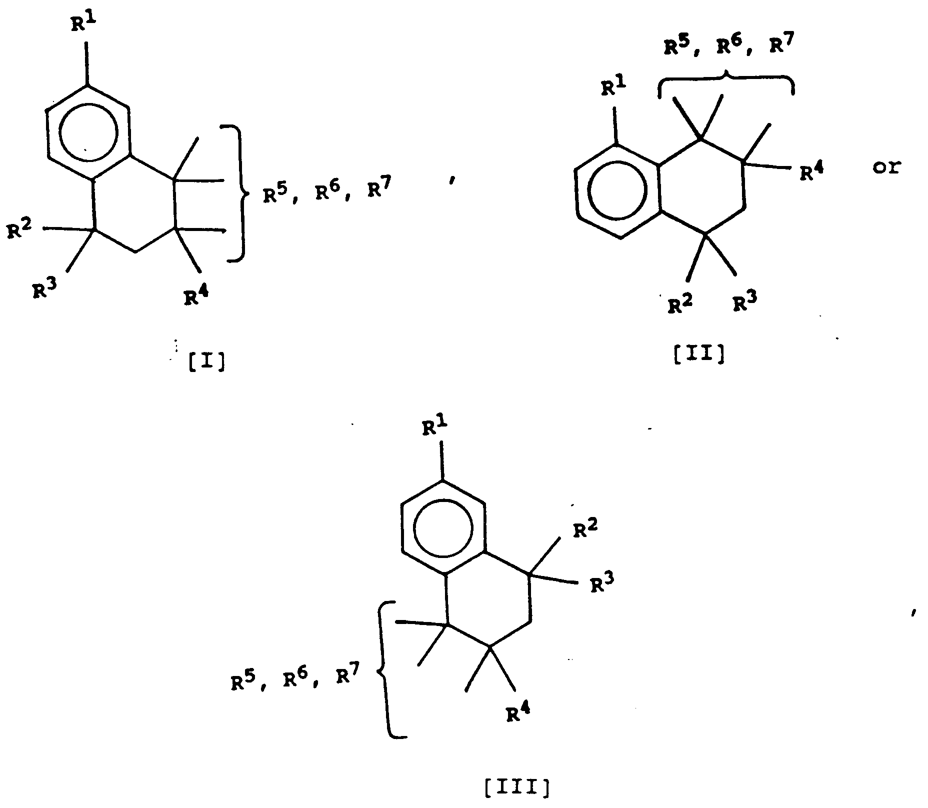 comprising contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, such as those compounds of the Formulas 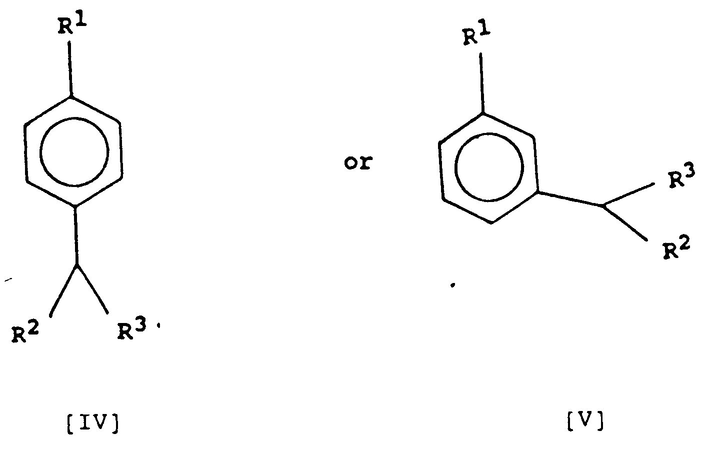 with an olefinic compound of the Formula 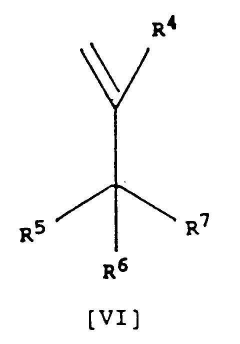 in the presence of a reagent of the Formula 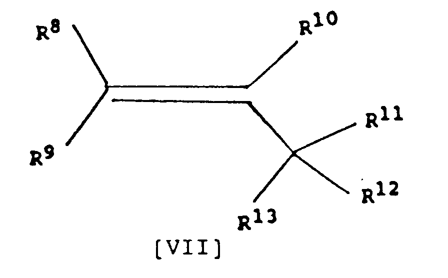 provided that said reagent has greater electron releasing properties than said olefinic compound of Formula VI, and further in the presence of an alkyl halide and a Lewis acid, wherein said process is carried out in the substantial absence of elemental iodine. In the above Formulas, R¹, R², R³, R⁴, R⁵, R⁶, R⁷, R⁸, R⁹, R¹⁰, R¹¹, R¹² and R¹³ are, independently, substituents that do not substantially interfere with a Friedel-Crafts-type alkylation reaction, provided that (i) R¹, R² and R³ are each other than H, (ii) no more than one of R⁵, R⁶ and R⁷ are H, (iii) no more than one of R⁸, R⁹ and R¹⁰ are H, and (iv) no more than one of R¹¹, R¹² and R¹³ are H. The process components further include a phase transfer agent.

The present invention also pertains to a process for producing polyalkyl tetrahydronaphthalenes, such as those represented by the Formulas 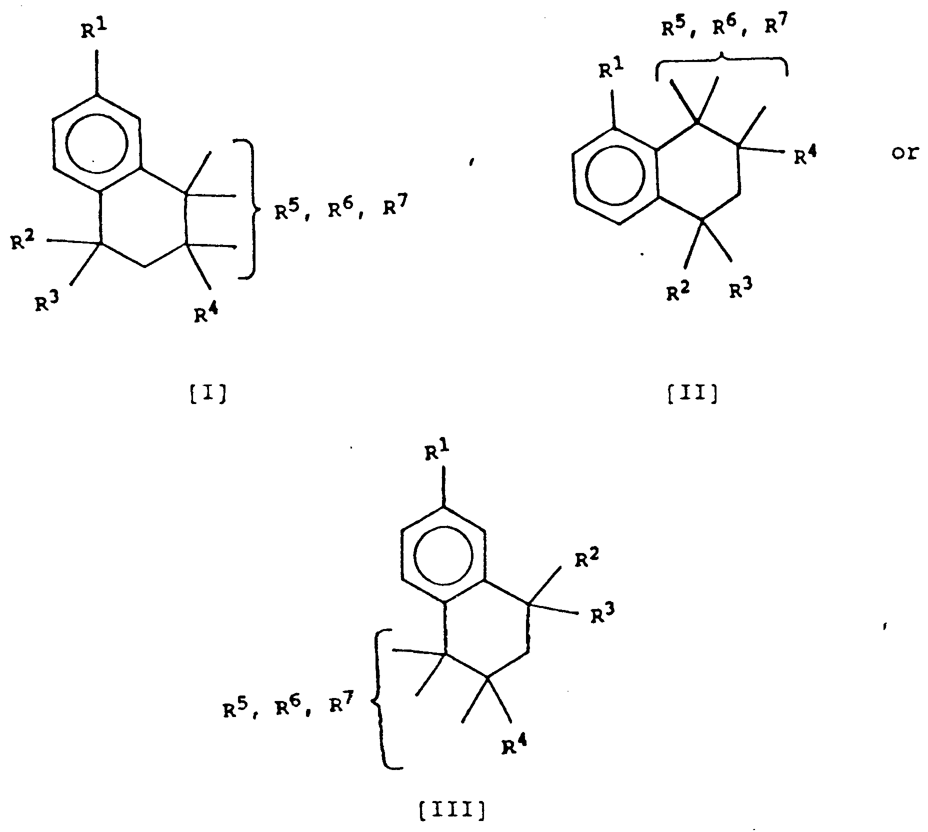 comprising contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, such as those compounds of the Formulas 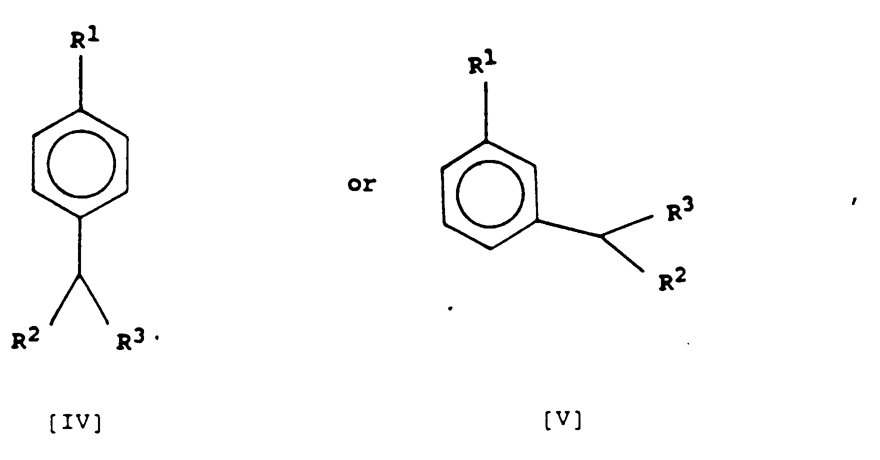 with an olefinic compound of the Formula 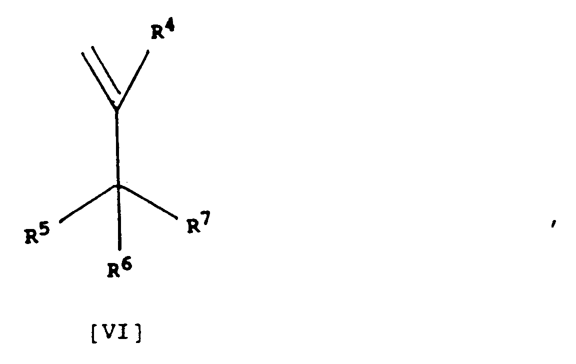 in the presence of a reagent of the Formula 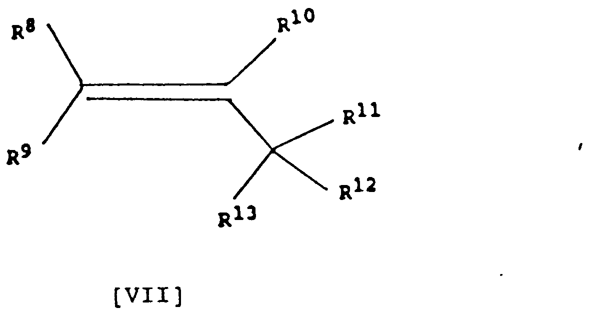 provided that said reagent has greater electron releasing properties than said olefinic compound Formula VI, and further in the presence of an alkyl halide, a Lewis acid, and a phase transfer agent, wherein R¹, R², R³, R⁴, R⁵, R⁶, R⁷, R⁸, R⁹, R¹⁰, R¹¹, R¹² and R¹³ are, independently, substituents that do not substantially interfere with a Friedel-Crafts-type alkylation reaction, provided that (i) R¹, R² and R³ are each other than H, (ii) no more than one of R⁵, R⁶ and R⁷ are H, (iii) no more than one of R⁸, R⁹ and R¹⁰ are H, and (iv) no more than one of R¹¹, R¹² and R¹³ are H.

The present invention further pertains to a process for producing polyalkyl tetrahydronaphthalenes, such as those represented by the Formulas 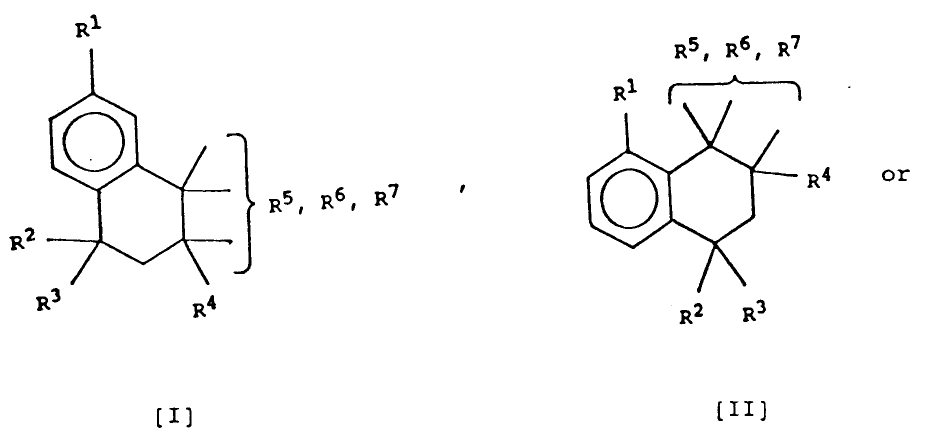 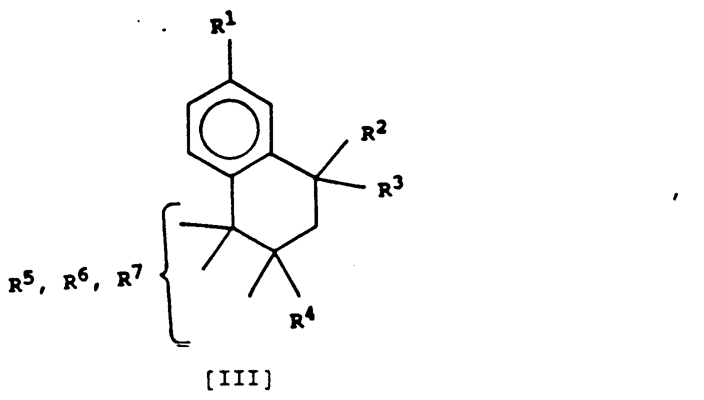 comprising contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, such as those compounds of the Formulas 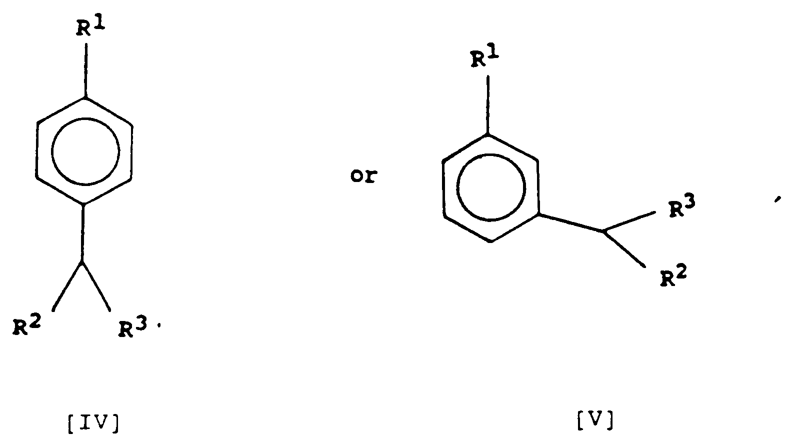 with an olefinic compound of the Formula 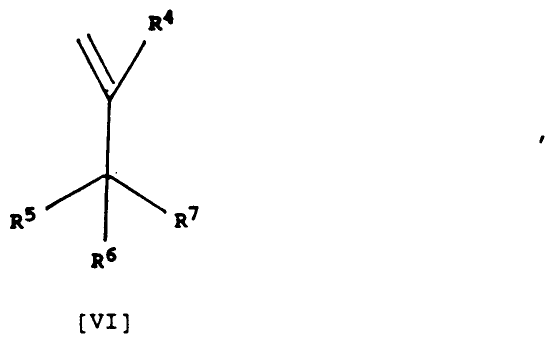 in the presence of an alkyl halide, a Lewis acid, and a phase transfer agent. In the above Formulas, R¹, R², R³, R⁴, R⁵, R⁶ and R⁷ are, independently, substituents that do not substantially interfere with a Friedel-Crafts-type alkylation reaction, provided that (i) R¹, R² and R³ are each other than H, and (ii) no more than one of R⁵, R⁶ and R⁷ are H.

Using the foregoing processes, one is able to produce a variety of alkyl-substituted tetrahydronaphthalene compounds for use as chemical intermediates and/or chemical products, particularly intermediates such as HMT, which is a compound of extreme importance to the fragrance industry.

### DETAILED DESCRIPTION OF THE INVENTION

As noted above, the present invention pertains to a novel and particularly useful process for the production of polyalkyl tetrahydronaphthalenes including, but not limited to, those of Formulas I, II or III:

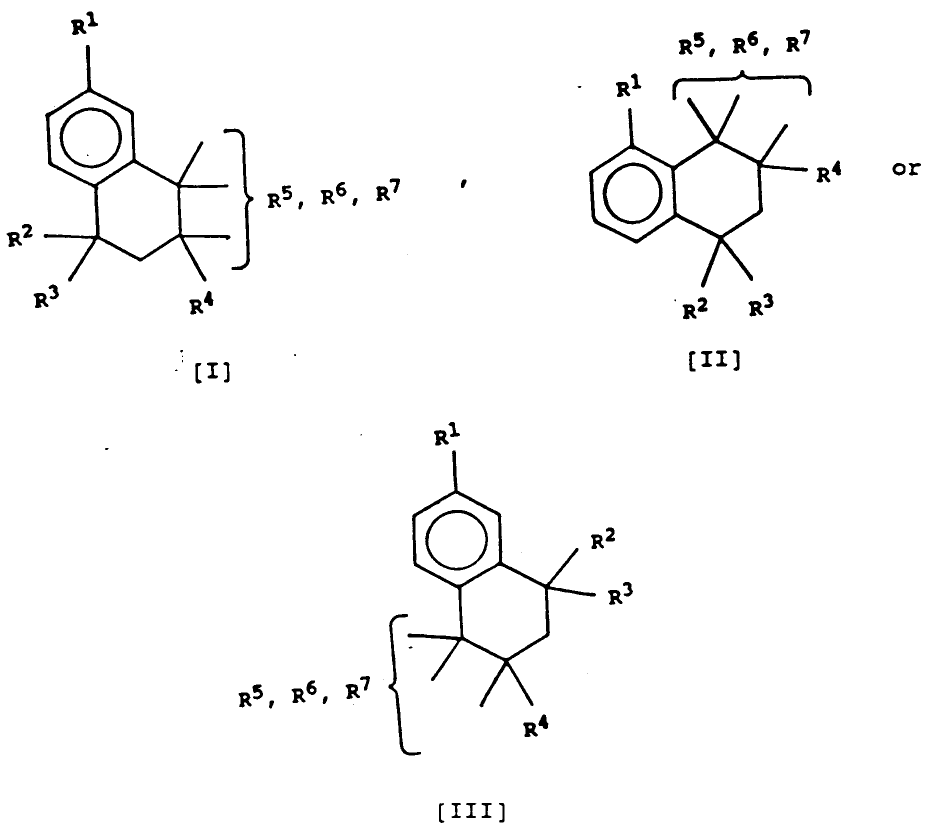

In the above Formulas, R¹, R², R³, R⁴, R⁵, R⁶ and R⁷ are defined, independently, as substituents that do not substantially interfere with a Friedel-Crafts-type alkylation reaction, provided that R¹, R² and R³ are each other than H, and no more than one of R⁵, R⁶ and R⁷ are H. The bracket notation as employed in Formulas I, II and III signifies that each of substituents R⁵, R⁶ and R⁷ can be present at any one of the attachment positions contained within the brackets, but not at more than one of these positions. In other words, the three attachment positions within the brackets are satisfied with an R substituent, one attachment position being satisfied with an R⁵ substituent, another with an R⁶ substituent, and a third with an R⁷ substituent. Suitable R¹, R², R³, R⁴, R⁵, R⁶ and R⁷ substituents will be readily apparent to those skilled in the art of Friedel-Crafts-type alkylation reactions. Such alkylation reactions and non-interfering substituents are discussed, for example, in George A. Olah, Friedel-Crafts and Related Reactions, Vols. 1 and 2 (Interscience Publishers, John Wiley and Sons, 1964) (hereinafter referred to as "Friedel-Crafts Reactions"), as well as in other journal and textbook references. The disclosures of Friedel-Crafts Reactions are incorporated herein by reference. Examples of suitable substituents include those wherein R¹, R² and R³ independently, are a C₁-C₃₀ straight chain, branched or cyclical alkyl, R⁴ is H or a C₁-C₃₀ straight chain, branched or cyclical alkyl, and R⁵, R⁶ and R⁷, independently, are H or a C₁-C₃₀ straight chain, branched or cyclical alkyl, provided that no more than one of R⁵, R⁶ and R⁷ are H. The alkyl is preferably a C₁-C₂₀, more preferably a C₁-C₁₀, and most preferably a C₁-C₅, alkyl. Preferably, the alkyl is a straight chain or branched alkyl. In a generally preferred embodiment, R¹, R² and R³, independently, are a C₁-C₅ straight chain or branched alkyl, R⁴ is H or a C₁-C₅ straight chain or branched alkyl, and R⁵, R⁶ and R⁷ are H or a C₁-C₅ straight chain or branched alkyl, provided that no more than one of R⁵, R⁶ and R⁷ are H.

In a most preferred embodiment, the polyalkyl tetrahydronaphthalenes are of the Formula I. The Formula I compounds are preferably:
o 1,1,3,4,4,6-hexamethyl-1,2,3,4-tetrahydronaphthalene (that is, HMT, a compound of Formula I wherein R¹, R², R³, R⁵, R⁶ and R⁷ are each methyl, and R⁴ is H);
o 6-ethyl-1,1,3,4,4-pentamethyl-1,2,3,4-tetrahydronaphthalene (that is, a compound of Formula I wherein R¹ is ethyl, and R², R³, R⁵, R⁶ and R⁷ are each methyl, and R⁴ is H);
o 6-tertiary-butyl-1,1,3,4,4-pentamethyl-1,2,3,4-tetrahydronaphthalene (that is, a compound of Formula I wherein R¹ is tertiary butyl, and R², R³, R⁵, R⁶ and R⁷ are each methyl, and R⁴ is H); and
o 6-n-propyl-1,1,3,4,4-pentamethyl-1,2,3,4-tetrahydronaphthalene (that is, a compound of Formula I wherein R¹ is n-propyl, and R², R³, R⁵, R⁶ and R⁷ are each methyl, and R⁴ is H).

The compounds of Formulas I, II and III are produced by contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, such substituted benzene compounds including, but not limited to, those of the Formulas IV or V 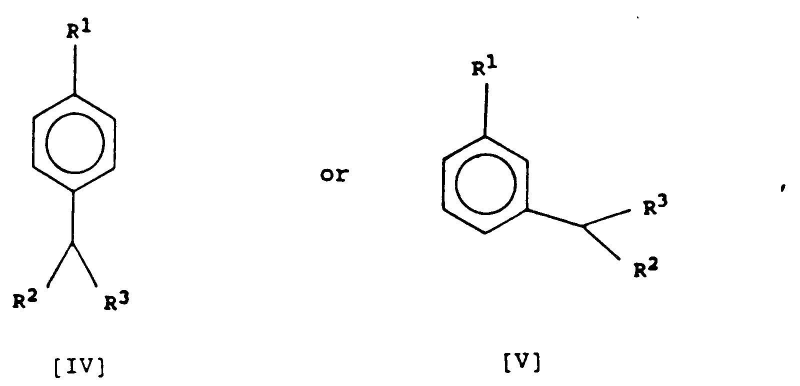 with an olefinic compound of the Formula VI

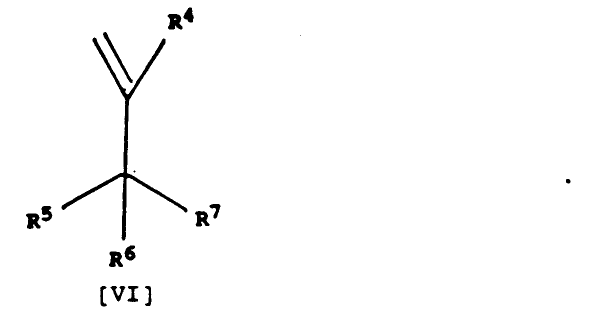

Contacting a benzene compound of Formula IV with an olefinic compound of Formula VI will yield the tetrahydronaphthalene compounds of Formula I. Alternatively, contacting a benzene compound of Formula V with an olefinic compound of Formula VI will yield the tetrahydronaphthalene compounds of Formulas II and III. The Formula I, II or III compounds may isomerize under the reaction conditions to also form compounds of one or more of the other Formula I, II or III compounds.

In the above Formulas IV, V and VI, R¹, R², R³, R⁴, R⁵, R⁶ and R⁷ are defined, independently, as previously described, that is, as substituents that do not substantially interfere with a Friedel-Crafts-type alkylation reaction, provided that R¹, R² and R³ are each other than H, and no more than one of R⁵, R⁶ and R⁷ are H. Suitable substituents are discussed in various journal and textbook references, such as Friedel-Crafts Reactions. Suitable substituents include those wherein R¹, R² and R³, independently, are a C₁-C₃₀ straight chain, branched or cyclical alkyl, R⁴ is H or a C₁-C₃₀ straight chain, branched a cyclical alkyl, and R⁵, R⁶ and R⁷, independently, are H or a C₁-C₃₀ straight chain, branched or cyclical alkyl, provided that no more than one of R⁵, R⁶ and R⁷ are H. The alkyl is preferably a C₁-C₂₀, more preferably a C₁-C₁₀, and most preferably a C₁-C₅, alkyl. Preferably the alkyl is a straight chain or branched alkyl.

With respect to the benzene compounds of Formulas IV and V, a generally preferred embodiment includes those compounds wherein R¹, R² and R³, independently, are a C₁-C₅ straight chain or branched alkyl. In a most preferred embodiment, the substituted benzene compounds are of Formula IV. The Formula IV compounds are preferably isopropyl toluene (that is, para-cymene, a compound of Formula IV wherein R¹, R² and R³ are each methyl), 1-ethyl-4-isopropylbenzene (that is, a compound of Formula IV wherein R¹ is ethyl, and R² and R³ are each methyl), 1-n-propyl-4-isopropylbenzene (that is, a compound of Formula IV wherein R¹ is n-propyl, and R² and R³ are each methyl), and 1-tertiary-butyl-4-isopropylbenzene (that is, a compound of Formula IV wherein R¹ is tertiary-butyl, and R² and R³ are each methyl).

In a generally preferred embodiment, the olefinic compounds of Formula VI include those compounds wherein R⁴ is H or a C₁-C₅ straight chain or branched alkyl, and R⁵, R⁶ and R⁷, independently, are a H or C₁-C₅ straight chain or branched alkyl, provided that no more than one of R⁵, R⁶ and R⁷, are H. A more preferable embodiment is wherein R⁴ is H or methyl. Of the Formula VI compounds, 3,3-dimethyl-1-butene (that is, neohexene, a compound of Formula VI wherein R⁴ is H, and R⁵, R⁶ and R⁷ are each methyl) and 2,3-dimethyl-1-butene (a compound of Formula VI wherein R⁴, R⁵ and R⁶ are each methyl, and R⁷ is H) are most preferred. As those skilled in the art would recognize, certain internal olefinic isomers of the terminal olefins of Formula VI can be employed in lieu of the Formula VI compounds. Such compounds are capable of rearranging by isomerization under the process conditions of the invention to form the Formula VI compounds believed to be required for tetrahydronaphthalene formation. A preferable internal olefinic isomer is 2,3-dimethyl-2-butene.

In accordance with the present invention, the compounds of Formulas IV or V are, in some embodiments, contacted with compounds of Formula VI in the presence of a reagent of the Formula 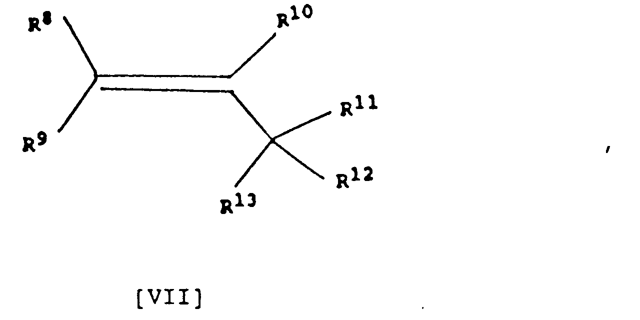 provided that said reagent has greater electron releasing properties than said olefinic compound, and in the presence of an alkyl halide and, in some embodiments, a hydrogen halide, a Lewis acid, and a phase transfer agent.

In the above Formula VII, R⁸, R⁹, R¹⁰, R¹¹, R¹² and R¹³ are defined, as previously described, that is, as substituents that do not substantially interfere with a Friedel-Crafts-type alkylation reaction, provided that no more than one of R⁸, R⁹ and R¹⁰ are H, and no more than one of R¹¹, R¹² and R¹³ are H. Suitable substituents include those wherein R⁸, R⁹, R¹⁰, R¹¹, R¹² and R¹³ are, independently, H or a C₁-C₃₀ straight chain, branched or cyclical alkyl. The alkyl is preferably a C₁-C₂₀, more preferably a C₁-C₁₀ and most preferably a C₁-C₅ alkyl. Preferably the alkyl is a straight chain or branched alkyl. In a most preferred embodiment, the Formula VII compound is 2,4,4-trimethyl-2-pentene (that is, diisobutylene-2, a compound of Formula VII wherein R⁸, R⁹, R¹¹, R¹² and R¹³ are methyl and R¹⁰ is H). The particular reagents defined in Formula VII have been found to be surprisingly effective hydride abstractors. These compounds are capable of preferentially carrying out the hydride abstraction function, rather than participating in the alkylation step. This results in a process which has a smaller amount of side reactions occurring, and thus a higher selectivity to and yield of the desired end product.

As noted above, the Formula VII compounds employed in a process of the invention must have greater electron releasing properties than the olefinic compounds VI also utilized in that process. The comparative electron releasing properties of the Formula VI and VII compounds will be readily apparent to those skilled in the art. As will be recognized, for example, any of the Formula VII compounds wherein the R⁸ through R¹³ substituents are selected from H or alkyl, will have greater electron releasing properties than any of the Formula VI compounds wherein the R⁴ through R⁷ substituents are also selected from H or alkyl. Accordingly, it is expected that the Formula VII compounds will function in the present process as the primary hydride abstracting agents, relieving the olefinic compounds VI of the task and enabling the Formula VI olefins to instead function as alkylating agents. In addition, by utilizing the Formula VII compounds in accordance with the present process, one will be employing in a non-productive reduction (hydride abstraction) step a more readily available, less expensive reagent VII, in lieu, at least in part, of the less readily available, more expensive alkyl halide compounds consumed in accordance with some prior art procedures. As a result, it is possible to avoid excessive formation of hydrogen halides and accumulation of such compounds in the product stream, an undesirable result associated with some art processes. Moreover, the potential for corrosion problems within the reaction system concomitant with the formation of the hydrogen halides may be lessened, and the need for complex procedures for the separation of the desired HMT-type products from the hydrogen halide by-products may be minimized.

Suitable alkyl halides include secondary alkyl halides, tertiary alkyl halides, propargyl halides and allyl halides. Exemplary secondary alkyl halides include isopropyl chloride, secondary-butyl chloride, secondary-amyl chloride, cyclohexyl chloride, and homologues thereof having fluorine, bromine or iodine atoms substituted for the chlorine atom, as well as various secondary alkyl dihalides. Examples of tertiary alkyl halides include tertiary-butyl chloride, tertiary-amyl chloride, 2-methyl-2-chloropentane, 3-methyl-3-chloropentane, as well as various other tertiary alkyl dihalides such as 1,8-dichloro-para-menthane, and homologues thereof having fluorine, bromine or iodine atoms substituted for the chlorine atom. Representative propargyl halides include propargyl chloride, 1-chloro-2-butyne, 1-chloro-2-pentyne, and homologues thereof having fluorine, bromine or iodine atoms substituted for the chlorine atom, as well as various propargyl dihalides. Suitable allyl halides include allyl chloride, 1-chloro-2-butene, 1-chloro-3-methyl-2-butene, 1-chloro-2-pentene, 1-chloro-2-hexene and homologues thereof having fluorine, bromine or iodine atoms substituted for the chlorine atom, as well as various allyl dihalides. Other suitable alkyl halides will be readily apparent to those skilled in the art. Of the foregoing alkyl halides, tertiary alkyl halides, and in particular tertiary-butyl chloride, tertiary-amyl chloride, 2-methyl-2-chloropentane, 3-methyl-3-chloropentane and 1,8-dichloro-para-menthane, are preferred. A most preferred alkyl halide is the tertiary alkyl halide which is tertiary-butylchloride. Although any of the halogen halides may be used in the embodiments of the invention which permit use of such compounds, preferable hydrogen halides are hydrogen chloride or hydrogen bromide, most preferably hydrogen chloride. Preferably, where there is a choice, an alkyl halide rather than a hydrogen halide is employed.

Any Lewis acid, that is, any non-protonic compound capable of accepting an electron pair, is suitable for use in the present process. Exemplary Lewis acids include metal halides such as aluminum halides (including aluminum chloride, aluminum bromide, aluminum iodide, monofluorodichloroaluminum, monobromodichloroaluminum and monoiododichloroaluminum), alkyl metal halides and alkyl metals. Alkyl metals and alkyl metal halides suitable for use as Lewis acids in the present process are disclosed, for example, in Kennedy, Joseph P., Carbocationic Polymerization, p. 221 (Wiley-Interscience Publishers (1982)), the disclosures of which are incorporated herein by reference. In the process of the present invention, aluminum halides are preferred. Of the aluminum halides, aluminum chloride and aluminum bromide, particularly aluminum chloride, are most preferred.

The reaction is carried out in the presence of a phase transfer agent. Suitable phase transfer agents include onium salts such as ammonium, phosphonium and sulfonium salts. Other phase transfer agents suitable for use in the present process will be readily apparent to those skilled in the art, once having been made aware of the present disclosure.

Examples of ammonium phase transfer agents include quaternary ammonium halides such as methyltrioctylammonium chloride, methyltrinonylammonium chloride, methyltridecylammonium chloride, hexadecyltrihexylammonium bromide, ethyltrioctylammonium bromide, didodecyldimethylammonium chloride, tetraheptylammonium iodide, dioctadecyldimethylammonium chloride, tridecylbenzylammonium chloride, ditricosylmethylammonium chloride, and homologues thereof having chlorine, fluorine, bromine or iodine atoms substituted for the enumerated halide atom. Also suitable for use in the present invention as phase transfer agents are tertiary amine compounds substituted with hydrocarbons, such as trioctyl amine, which, under the conditions of the subject process may be converted to form quaternary ammonium salts. Trioctyl amine is commercially available from Sherex Co., located in Dublin, Ohio, under the tradename Adogen-364™.

Exemplary phosphonium phase transfer agents include quaternary phosphonium halides such as tributyldecylphosphonium iodide, triphenyldecylphosphonium iodide, tributylhexadecylphosphonium iodide, and homologues thereof having chlorine, fluorine or bromine atoms substituted for the iodine atom. In addition, trisubstituted phosphine compounds substituted with hydrocarbons, such as tri-n-butyl phosphine, may be converted to quaternary phosphonium salts under the present reaction conditions, and as such, are also suitable for use in the subject process as phase transfer agents.

Representative sulfonium phase transfer agents include ternary sulfonium halides such as lauryldimethylsulfonium iodide, lauryldiethylsulfonium iodide and tri(n-butyl)sulfonium iodide, and homologues thereof having chlorine, fluorine or bromine atoms substituted for the iodine atom. In addition, disubstituted sulfur compounds substituted with hydrocarbons may be converted to ternary sulfonium salts under the present reaction conditions, and as such, are also suitable for use in the subject process as phase transfer agents.

These and other suitable phase transfer agents are described, for example, in Napier et al., U.S. Patent No. 3,992,432 and in Kondo et al., Synthesis, pp. 403-404 (May 1988), the disclosures of which are incorporated herein by reference.

Preferable phase transfer agents are ammonium or sulfonium salts, particularly quaternary ammonium or ternary sulfonium halides. Most preferred are quaternary ammonium halides, particularly methyltrioctylammonium chloride (referred to herein as "MTOAc"), and a mixture of methyltrioctylammonium chloride and methyltridecylammonium chloride. The latter mixture is marketed under the tradename Adogen-464™, by Sherex Co., located in Dublin, Ohio.

In general, the molar proportions of the reagents employed in the present process can be varied over a relatively wide range. However, where phase transfer agents are employed in the process, it is important, for the best results, to maintain a ratio of less than one mole of phase transfer agent per mole of Lewis acid. Preferably, the molar ratio is about 0.8 to 1.0, more preferably 0.5 to 1.0, phase transfer agent to Lewis acid. It should be noted that some phase transfer agents sold commercially are sold in an impure form. Such impurities generally comprise water or an alcohol species. Water and alcohol, as well as other impurities, will react adversely with the Lewis acid, thereby lowering the amount of active Lewis acid available for the process of the present invention. Accordingly, where the phase transfer agent added contains such impurities, the amount of Lewis acid should thus preferably be increased to account for these impurities. In such a situation the ratio of transfer agent to Lewis acid might be about 0.3 to 1.0. Such impure agent-containing mixtures are referred to herein as mixtures in an "impure form".

It is preferable to use a mixture of olefinic compound VI, alkyl halide and hydrogen halide (if employed), and hydride abstracting reagent VII (if employed), wherein these components are present in a molar range of about 1.0 to about 5.0 moles of olefin VI per mole of combined halides plus any reagent VII. More preferably, the olefin VI, and the combined halides plus reagent VII are present in nearly equimolar amounts, that is, about 1.0 mole of olefin VI per mole of combined halides plus reagent VII.

Preferably, the substituted benzene compound is present in a range of about 0.5 to about 10 moles per mole of olefin VI. More preferably, the substituted benzene compound is present in a range of about 0.5 to about 5.0 per mole of olefin VI.

In a most preferred embodiment, each of the benzene compound, olefin VI, and the combination of alkyl halide, hydrogen halide plus hydride abstracting reagent VII, are present nearly in equimolar amounts, that is, about 1.0 mole of benzene compound, to about 1.0 mole of olefin VI, to about 1.0 mole of combined halides plus hydride abstracting reagent VII.

The amount of Lewis acid utilized is preferably in the range of about 2% to about 10% by weight of the Lewis acid based on the combined weight of the substituted benzene, olefin VI, alkyl halide, and hydrogen halide (if employed) plus hydride abstracting reagent VII (if employed).

As noted above, in certain embodiments, the present process must be conducted in the substantial absence of elemental iodine (I₂). By "substantial absence", it is meant that only a deminimus amount of iodine (such as, for example, less than 1% by weight of I₂ based on the weight of the Lewis acid), if any, is present in the reaction medium. Preferably, in the embodiments which require a substantial absence of iodine, the reaction medium is devoid of any elemental iodine.

The reaction is generally carried out using a solvent, although, if desired, substituted benzene, one of the starting materials, may be employed in large excess in lieu of an additional solvent. A number of different solvents may be utilized in the present invention, including halogenated and unhalogenated aliphatic, alicyclic and aromatic hydrocarbon solvents.

Where the process is run in the absence of a phase transfer agent, and the olefinic compound of Formula VI is one wherein R⁵, R⁶ and R⁷ are other than H, such halogenated aliphatic, halogenated alicyclic and halogenated aromatic hydrocarbon solvents are preferred, for reasons of increased yield. Representative of the halogenated solvents are the aliphatic solvents methylene chloride, chloroform, carbon tetrachloride, ethylene chloride, ethylidene chloride, 1,1,1-trichloroethane, 1,1,2-trichloroethane, 1,1,2,2-tetrachloroethane, 1,2-dichloroethylene, trichloroethylene, tetrachloroethylene, 1,2,3-trichloropropane, amyl chloride, and ethylene bromide, and the aromatic solvents monochlorobenzene, ortho-dichlorobenzene, bromobenzene and fluorobenzene.

Where a phase transfer agent is employed in connection with the subject process or the olefinic compound of Formula VI is one wherein one of R⁵, R⁶ and R⁷ are H, the unhalogenated aliphatic, unhalogenated alicyclic and unhalogenated aromatic hydrocarbon solvents are preferred, for reasons of increased yield, safety and/or process engineering. Exemplary of the unhalogenated solvents are the aliphatic solvents n-hexane, n-heptane and n-octane, the alicyclic solvent cyclohexane, and the aromatic solvents benzene, toluene, ethylbenzene and xylene. Particularly preferred for reasons of yield, safety and/or process engineering are the unhalogenated aliphatic and unhalogenated alicyclic hydrocarbons. Other suitable halogenated and unhalogenated solvents are described, for example, in U.S. Patent Nos. 4,284,818, 3,856,875 and 3,379,785, the disclosures of which are incorporated herein by reference.

The alkylation reaction of the invention can be carried out in any suitable vessel which provides efficient contacting between the Lewis acid and the other reactants. For simplicity, a stirred batch reactor can be employed. Although stirring is recommended to provide efficient contact between reactants, it has been found that with the addition of the phase transfer agent pursuant to some embodiments of the present invention, the Lewis acid is able to solubilize rather quickly and cleanly, thereby obviating the need for the stringent stirring requirements of many of the art processes utilized to produce HMT. The reaction vessel used should be resistant to the possibly corrosive nature of the catalyst. Glass-lined vessels are suitable for this purpose, as well as other vessel materials well known in the art.

The reagents of the present process may be added in any order, although where the process is carried out with a phase transfer agent, a preferred mode is to add the solvent, the Lewis acid and the phase transfer agent first, allow sufficient time for the Lewis acid to become substantially dissolved in the solvent, and then add the remaining reagents. Generally, 15 to 30 minutes are needed for the Lewis acid to become substantially dissolved in the solvent.

Ideally, the reaction is carried out at temperatures ranging from about -30°C to about 50°C, preferably at temperatures ranging from about -10°C to about 40°C, and most preferably at temperatures ranging from about 0°C to about 30°C.

The pressure at which the reaction is carried out is not critical. If the reaction is carried out in a sealed vessel, autogenous pressure is acceptable, although higher or lower pressures, if desired, may be employed. The reaction can also be carried out at atmospheric pressure in an open reaction vessel, in which case the vessel is preferably equipped with a moisture trap to prevent significant exposure of Lewis acid to moisture. The reaction can take place in an oxygen atmosphere, or an inert atmosphere as in the presence of a gas such as nitrogen, argon and the like, the type of atmosphere also not being critical.

Reaction time is generally rather short and is often dictated by the kind of equipment employed. Sufficient time must be provided, however, for thorough contacting of the substituted benzene compound, the olefinic compound, the Lewis acid and the phase transfer agent. Generally the reaction proceeds to completion in about 1 to about 7 hours.

Product can be recovered by first quenching the reaction mixture in cold water or on crushed ice, preferably on ice, and then processing the mixture in the usual manner for Friedel-Crafts reactions to extract the desired alkyl-substituted tetrahydronaphthalene compounds. Suitable extraction protocol is described, for example, in Friedel-Crafts Reactions. Typically, following quenching and the resultant phase separation, the organic layer is washed an additional time with water to aid in removal of the Lewis acid. One or more additional washings can be carried out with dilute alkali solution to further aid Lewis acid removal. Pure product can then be recovered by subjecting the washed reaction mixture to reduced pressure fractional distillation.

The polyalkyl tetrahydronaphthalenes prepared in accordance with the processes of the invention, as herein-before indicated, may, for example, be acylated to obtain acylated polyalkyl tetrahydronaphthalenes having very fine, musk-like fragrances, a characteristic which renders them highly valuable for use in the perfumery industry. Such products, acylated or otherwise, may alternatively or additionally have utility in the pharmaceutical and/or agrochemical industries, either as intermediates or as end products, as generally discussed in French Patent Publication No. 2601670, and U.S. Patent No. 4,551,573. The acylation process may be carried out using conventional methods, such as by reacting the polyalkyl tetrahydronaphthalene with an acyl halide or acid anhydride in the presence of an acid-acting catalyst. Suitable acylation methods are well known in the art and are disclosed, for example, in U.S. Patent No. 4,284,818. Examples of acylated polyalkyl tetrahydronaphthalenes include 7-acetyl-1,1,3,4,4,6-hexamethyl-1,2,3,4-tetrahydronaphthalene, 7-acetyl-1,1,3,4,4-pentamethyl-6-ethyl-1,2,3,4-tetrahydronaphthalene, 7-acetyl-1,1,3,4,4-pentamethyl-6-n-propyl-1,2,3,4-tetrahydronaphthalene, and 7-acetyl-1,1,3,4,4-pentamethyl-6-tertiary-butyl-1,2,3,4-tetrahydronaphthalene.

The present invention is further described in the following Examples. These Examples are not to be construed as limiting the scope of the appended Claims.

In each Example, the reaction flasks were equipped with a condenser, mechanical stirrer, addition funnel and thermocouple/temperature controller connected to an automatic laboratory jack. The flasks were cooled, when necessary, with a dry ice/isopropanol bath. The flask contents were continuously stirred throughout the reaction.

Results were analyzed on both polar and non-polar gas chromatography columns. All gas chromatography analyses were carried out on capillary columns using a weight percent internal standard method of analysis. Structure identifications were assigned based on GCMS fragmentation patterns compared to standards.

Examples 1, 3, 4, 8, 9, 12, 13, 17, 21 and 24 are provided for comparative purposes only, and do not illustrate processes of the present invention. Specifically, Example 1 was carried out substantially in accordance with the procedures set forth in Wood et al., U.S. Patent No. 3,856,875. Examples 3 and 4 were carried out substantially in accordance with the procedures set forth in Sato et al., U.S. Patent No. 4,284,818, except that methyltrioctylammonium chloride and Adogen-464™, respectively, were added. Example 8 was also carried out substantially in accordance with the procedures set forth in U.S. Patent No. 4,284,818. Example 9 is similar to Example 8, except that methyltrioctylammonium chloride was added. Examples 13 and 17 were carried out substantially in accordance with the procedures set forth in U.S. Patent Nos. 4,284,818 and 3,856,875, respectively. Example 21 was carried out substantially in accordance with the procedures set forth in U.S. Patent No. 4,284,818. Example 24 is similar to Example 21 except that the reagents were added at twice the rate. Examples 5, 6, 7, 11, 14-16, 18-20, 22 and 23 are examples of processes of the present invention.

### Examples

### Example 1 (Comparative Example)

An oven-dried, 100 ml, four-necked, round bottom flask was charged with 1,2-dichloroethane (10.10 g), and anhydrous aluminum chloride (0.962 g). Next, a mixture of para-cymene (31.67 g), tertiary-butyl chloride (10.61 g) and neohexene (8.84 g) was added to a 60 ml addition funnel connected to the flask. Addition of the mixture through the funnel was carried out over a period of about 2 hours. During this time the temperature of the flask was maintained at about -8°C. The mixture was allowed to stir an additional 3 hours with the temperature during this period held between about -6 to about -3°C. The reaction was then quenched with deionized water (20 ml) and the organic phase separated and washed with, in order, 5% HCl, 10% Na₂CO₃ and 50% (that is, half-saturated) brine solution. Each aqueous wash was individually extracted with ethyl ether, and the ether layers combined with the organic phase. The organics were then dried over K₂CO₃, filtered, and evaporated to yield a crude product (37.46 g) containing 34.46 weight % HMT (58.30% molar yield of HMT based on the amount of neohexene charged).

The Example was carried out substantially in accordance with the procedure set forth in Wood et al., U.S. Patent No. 3,856,875.

### Example 3 (Comparative Example)

A 50 ml, three-necked, round bottom flask was charged with cyclohexane (9.55 g), tertiary-butyl chloride (4.81 g) and para-cymene (12.57 g) and cooled to about 0°C. A 60 ml addition funnel was charged with neohexene (3.82 g, 97% pure) and connected to the flask. To the flask was then added anhydrous (0.914 g) and methyltrioctylammonium chloride (1.37 g). Addition of the funnel neohexene was commenced and proceeded over a 3-hour period while the temperature of the flask was maintained at about 0°C. The flask ingredients were stirred an additional 3.5 hours at a temperature of about 0°C, the reaction quenched and the mixture worked up as previously described to yield a crude product (15.26 g) containing 42.3 weight % HMT (67.8% molar yield of HMT based on the amount of neohexene charged).

### Example 4 (Comparative Example)

A 100 ml, four-necked, round bottom flask was charged with Adogen-464™ (1.515 g) and cyclohexane (19.10 g) and cooled to about 16°C. Next, anhydrous aluminum chloride (1.43 g) was added to the flask and the mixture stirred for about 0.5 hours at about 16°C. A mixture of para-cymene (25.14 g) and tertiary-butyl chloride (8.40 g) was then added to the flask. Immediately, neohexene addition was started using a syringe pump, while the flask temperature was adjusted to and maintained at about 0°C. A total of 7.64 g neohexene (97% pure) added over a period of about 1.5 hours. After stirring an additional 0.5 hours, the reaction was quenched with water (10 ml) and worked up as previously described to yield a crude product (30.00 g) containing 36.22 weight % HMT (57.1 molar yield of HMT based on the amount of neohexene charged).

### Example 5

A 100 ml, four-necked, round bottom, flask was charged with cyclohexane (15.28 g), Adogen-464™ (1.214 g) and anhydrous aluminum chloride (1.139 g). The mixture was cooled to about 16°C and stirred for about 0.5 hours. The flask was then cooled over a 3-minute period to about 3°C as neohexene addition was started from a syringe pump. At the same time, para-cymene (20.11 g) and tertiary-butyl chloride (0.67 g) were charged to the flask, and diisobutylene-2 addition was started from a second syringe pump. After 90 minutes of neohexene (6.14 g) addition, and 87 minutes of diisobutylene-2 (4.51 g) addition, the syringe pumps were turned off and the flask mixture stirred an additional 10 minutes. The reaction was then quenched with deionized water and the organic phase separated and washed with, in order, 5% HCl, 10% Na₂CO₃ and 50% brine solution. Each aqueous wash was individually extracted with ethyl ether, and the ether layers combined with the organic phase. The organics were then dried over K₂CO₃, filtered, and evaporated to yield a crude product (23.98 g) containing 42.60 weight % HMT (65.81% molar yield of HMT based on the amount of neohexene charged).

### Example 6

A 100 ml, four-necked round bottom flask was charged with cyclohexane (15.28 g), Adogen-464™ (1.219 g) and anhydrous aluminum chloride (1.219 g). The mixture was cooled to about 16°C, and stirred for about 0.5 hours. Addition of neohexene (6.14 g), para-cymene (20.11 g), tertiary-butyl chloride (0.34 g) and diisobutylene-2 (4.51 g) was carried out substantially as described in Example 5. The reaction was allowed to stir an additional 10 minutes and then quenched with deionized water (15 ml). The organic phase was washed with, in order, 5% HCl, 10% Na₂CO₃ and 50% brine solution. Each aqueous layer was individually extracted with ethyl ether, and the ether layers combined with the organic phase. The organics were then dried over K₂CO₃, filtered and evaporated to yield a crude product (22.64 g) containing 44.44 weight % HMT (64.82% molar yield of HMT based on the amount of neohexene charged.)

### Example 7

A 100 ml, four-necked, round bottom flask was charged with Adogen 464™ (1.528 g), cyclohexane (19.10 g) and anhydrous aluminum chloride (1.432 g) with initial cooling to 16°C. Neohexene (7.53 g) and diisobutylene-2 (8.02 g) were added to the flask mixture over a period of about 90 minutes, and about 87 minutes, respectively. Para-cymene (25.14 g) and tertiary-butyl chloride (1.68 g) were added directly to the flask after neohexene had been added for about 2 minutes. Stirring of the flask mixture was continued for an additional 10 minutes at which time deionized water (15 ml) was added to quench the reaction. The reaction mixture was worked up as previously described to yield a crude product (30.11 g) containing 43.37 weight % HMT (67.63% molar yield of HMT based on the amount of neohexene charged).

Examples 3 and 4 are used for comparative purposes and illustrate two different ways of carrying out reactions similar in nature to Examples 5, 6 and 7, which are carried out in accordance with the teachings of this invention. Examples 3 and 4 use only tertiary butyl chloride as the hydride abstracting agent, while 5, 6 and 7 replace a portion of the tertiary butyl chloride with the less expensive olefin diisobutylene-2. This resulted in at least a maintaining of the molar yield of HMT compared to Examples 3 and 4, although one of the key ingredients (tertiary butyl chloride) had been partially replaced.

### Example 8 (Comparative Example)

A 100 ml four-necked round bottom flask was charged with cyclohexene (19.10 g) and cooled to 20°C with a dry ice/isopropanol bath. Anhydrous aluminum chloride (1.803 g) was added, with stirring, to the cyclohexene. Next, a mixture containing para-cymene (25.13 g), 2,3-dimethyl-1-butene (7.63 g), and tertiary-butyl chloride (9.52 g) was added to the flask over a period of about 2 hours and 45 minutes. At 3 hours, the reaction was quenched with 10 ml of deionized water. The organic phase was washed with, in order, 5% HCl, 10% Na₂CO₃, and 50% (half-saturated) brine solution. The aqueous layers were individually extracted with ethyl ether, and the ether layers combined with the organic phase. The organics were then dried over K₂CO₃, filtered, and evaporated to yield a crude product (29.10 g) containing 28.57 weight % HMT (42.46% molar yield of HMT based on the amount of 2,3-dimethyl-1-butene charged).

### Example 9 (Comparative Example)

A 100 ml four-necked round bottom flask was charged with cyclohexene (19.10 g). To this was added methyltrioctylammonium chloride (2.735 g) and anhydrous aluminum chloride (1.803 g). The mixture was cooled to 20°C and allowed to stir for thirty minutes. A mixture of para-cymene (25.13 g), 2,3-dimethyl-1-butene (7.63 g), and tertiary-butyl chloride (9.52 g) was then added to the flask over a period of three hours. When the addition was complete, the reaction was quenched with 15 ml of deionized water. The organic phase was washed with, in order, 5% HCl, 10% Na₂CO₃, and 50% brine solution. Each aqueous layer was extracted with ethyl ether, and the ether layer combined with the organic phase. The organics were then dried over K₂CO₃ and evaporated to yield a crude product (33.78 g) containing 29.22 weight % HMT (50.40% molar yield of HMT based on the amount of 2,3-dimethyl-1-butene charged).

### Example 11

A 100 ml four-necked round bottom flask was charged with cyclohexane (19.10 g) and methyltrioctylammonium chloride (2.73 g), and cooled to 20°C. Anhydrous aluminum chloride (1.803 g) was added and the mixture was stirred at 20°C for 0.5 hours. A solution containing a mixture of para-cymene (25.13 g), 2,3-dimethyl-1-butene (7.63 g), tertiary-butyl chloride (0.95 g), and diisobutylene-2 (10.38 g) was added to the flask over a 3 hour period while maintaining the temperature at 20°C. The reaction was then quenched with deionized water (15 ml). The organic phase was washed with, in order, 5% aqueous HCl, 10% Na₂CO₃ and 50% brine solution. The aqueous layers were each extracted with ethyl ether, the ether layers combined with the initial organic phase, dried over K₂CO₃, filtered and evaporated to give a crude product (33.77 g) containing 34.19 weight % HMT (58.96% molar yield of HMT based on the amount of 2,3-dimethyl-1-butene charged).

### Example 13 (Comparative Example)

Cyclohexane (19.10 g) and anhydrous aluminum chloride (1.80 g) were added to a 50 ml four-necked round bottom flask. The flask was charged with a well-stirred mixture of para-cymene (25.13 g, 98%), neohexene (7.96 g, 97%) and tertiary-butyl chloride (9.52 g, 98%), over about a three-hour period. During the addition process, the temperature of the flask was maintained at about 17-20°C with the aid of the temperature controller, automatic laboratory jack, and dry ice/isopropanol bath. During the first two hours of addition, a large amount of orange solids appeared on the sides of the flask. During the last hour of addition, these solids went back into solution. After addition was completed, the reaction was quenched with ice water (20 ml), and the resultant product washed with, in order, 5% aqueous HCl, 10% aqueous Na₂CO₃, and water. All aqueous layers were individually extracted with ether, the ether layers combined with the initial organic phase and evaporated to yield a crude product (30.75 g) containing 24.55 weight % of 1,1,3,4,4,6-hexamethyl-1,2,3-4-tetrahydronaphthalene (HMT) (7.55 g, 38.1% molar yield of HMT based on the amount of neohexene charged).

This Example was carried out substantially in accordance with the procedure set forth in Sato et al., U.S. Patent No. 4,284,818, example #8, with the exception that the 2,3-dimethyl-1-butene has been replaced with neohexene.

This Example shows that neohexene cannot be substituted for 2,3-dimethyl-1-butene when the reaction is carried out as described in Sato et al. without a loss in product yield. This is in direct agreement with the findings of Wood et al., U.S. Patent No. 3,856,875, which lists hydrocarbon solvent, such as hexane, as unsatisfactory.

### Example 14

A 50 ml three-necked round bottom flask was charged with cyclohexane (9.55 g), anhydrous aluminum chloride (0.912 g), methyltrioctylammonium chloride (1.37 g, 97% pure) and stirred for about 5 minutes. To the flask was then added a mixture of neohexene (3.82 g, 97% pure), tertiary-butyl chloride (4.81 g, 99% pure), and para-cymene (12.57 g, 96% pure). During the addition process, the temperature of the flask was maintained at about 20°C. Over a period of about 3 hours, the resultant product was then treated as in Example 13, to yield a crude product (14.96 g) containing 30.8 weight % HMT (48.5% molar yield of HMT based on the amount of neohexene charged).

This Example was carried out within the procedure of Example 13 of this disclosure, with the exception that a phase transfer agent, methyltrioctylammonium chloride, was added in accordance with the teachings of this invention. This resulted in a approximate yield increase of 27% over Example 13.

### Example 15

A 50 ml three-necked round bottom flask was charged with cyclohexane (9.55 g), tertiary-butyl chloride (4.81 g) and para-cymene (12.57 g) and cooled to 0°C. Next, anhydrous aluminum chloride (0.914 g) and methyltrioctylammonium chloride (1.37 g) were added to the flask. Neohexene (3.82 g, 97% pure) was then added over a period of about three hours, with the temperature of the flask being maintained at about 0°C, then treated as described in Example 13 to yield a crude product (15.26 g) containing 42.3 weight % HMT (67.8% molar yield of HMT based on the amount of neohexene charged).

This Example is an example of the present invention demonstrating an alternative mode of reagent addition.

### Example 16

A 100 ml four-necked round bottom flask was charged with Adogen-464™ (1.515 g), cyclohexane (19.10 g) and the flask was cooled to about 16°C. The anhydrous aluminum chloride (1.43 g) was then added and the mixture stirred for about 0.5 hours while maintaining the flask at about 16°C. Next, a mixture of para-cymene (25.14 g) and tertiary-butyl chloride (8.40 g) was added to the flask. Immediately thereafter, neohexene addition was started (via syringe pump), while the flask temperature was adjusted to and maintained at about 0°C. A total of 7.64 g neohexene (97% pure) was added over a period of about 1.5 hours. After stirring an additional 0.5 hours, the reaction was then quenched with water (10 ml) and the resultant product treated as previously described, to yield a crude product (30.00 g) containing 36.22 weight % HMT (57.1% molar yield of HMT based on the amount of neohexene charged).

This Example is an example of the present invention, demonstrating the use of the commercially available phase transfer agent Adogen 464™.

### Example 17 (Comparative Example)

A 100 ml three-necked round bottom flask was charged with 1,2 dichloroethane (10.10 g) and anhydrous aluminum chloride (0.962 g). The flask was cooled to about -8°C. An addition funnel was charged with para-cymene (31.67 g), tertiary-butyl chloride (10.61 g) and neohexene (8.84 g, 97% pure), and the funnel reagents were added over a period of about 2 hours while maintaining the temperature of the flask at about -6 to -8°C. After 3 hours of additional stirring while maintaining that same temperature, the reaction was quenched with ice water (20 ml), and treated as described in Example 13 to yield a crude product (37.46 g) containing 35.63 weight % HMT (60.6% molar yield of HMT based on the amount of neohexene charged).

This Example was carried out substantially in accordance with Wood et al., U.S. Patent No. 3,856,875.

### Example 18

A 50 ml three-necked round bottom flask was charged with anhydrous aluminum chloride (0.50 g), Adogen464™ (0.83 g) and 1,2-dichloroethane (7.51 g). The mixture was cooled to about -8°C. An addition funnel was charged with para-cymene (15.20 g), tertiary-butyl chloride (5.23 g) and neohexene, (4.20 g, 97% pure), and the funnel reagents added over a period of about 1.75 hours while maintaining a temperature of about -6 to -8°C. This temperature was maintained for an additional three hours while stirring was continued. The reaction was then quenched with ice water (10 ml) and treated as described in Example 13 to yield a crude product (17.84 g) containing 34.3 weight % HMT (58.5% molar yield of HMT based on the amount of neohexene charged).

This Example was carried out in the same fashion as Example 17, with the exception that the phase transfer agent Adogen 464™ was added. This shows that under satisfactory solvent conditions as described in Wood et al., U.S. Patent No. 3,856,875, no yield advantage is noted upon addition of a phase transfer agent.

### Example 19

A 50 ml three-necked round bottom flask was charged with cyclohexane (9.55 g), para-cymene (12.57 g), and 1,8-dichloro-para-menthane (5.43 g). The reaction was cooled to about 0°C and anhydrous aluminum chloride (0.913 g) and methyltrioctylammonium chloride (1.368 g) were quickly added. Immediately neohexene (3.48 g, 97% purity) was added via syringe pump over a period of about 2 hours at about 0°C. After about 4.5 hours additional stirring while maintaining that same temperature, the reaction was quenched with water (10 ml) and treated as described in Example 13 to yield a crude product (18.15 g) containing 25.86 weight % HMT (54.1% molar yield of HMT based on the amount of neohexene charged).

This Example describes the use of the dihalide 1,8-dichloro-para-menthane in accordance with the teachings of this invention.

### Example 20

A 100 ml four-necked round bottom flask was charged with trioctylamine (1.23 g), cyclohexane (19.10 g), and anhydrous aluminum chloride (1.43 g). The flask was cooled to about 20°C and stirred for about 0.5 hours. Next, para-cymene (25.14 g) was added to the flask. Following the para-cymene addition, tertiary-butyl chloride (7.70 g) and neohexene (7.09 g, 97% pure) were added independently at 20°C over a period of about 1 hour and 2 hours, respectively. Addition of neohexene was such that the initial rate was faster than the final rate, by a factor of about 2. The tertiary-butyl chloride addition rate was linear. One hour after the neohexene addition was complete, the reaction was quenched with water (20 ml) and worked up as described in Example 13 to yield a crude product (29.15 g) containing 28.0 weight % HMT (46.2 mole % yield of HMT based on the amount of neohexene charged).

Example 20 describes the use of trioctylamine as the phase transfer agent in accordance with the teachings of this invention.

### Example 21 (Comparative Example)

A 100 ml, four-necked, round bottom flask was charged with cyclohexane (19.10 g) and cooled to 20°C with a dry ice/isopropanol bath. Anhydrous aluminum chloride (1.803 g) was added, with stirring, to the cyclohexane. An addition funnel containing a mixture of para-cymene (25.13 g), 2,3-dimethyl-1-butene (7.63 g), and tertiary-butyl chloride (9.52 g) was connected to the flask and the mixture was added over a period of 2.77 hours. At 3 hours, the reaction was quenched with 10 ml of deionized water. The organic layer was washed, in order, with 5% HCl, 10% Na₂CO₃, and a 50% (that is, half-saturated) brine solution. Each aqueous wash was individually extracted with ethyl ether, and the ether layers combined with the organic phase. The organics were then dried over K₂CO₃, filtered, and evaporated to give a crude product (29.10 g) containing 28.57 weight % HMT (42.46 % molar yield of HMT based on the amount of 2,3-dimethyl-1-butene charged).

This Example was carried out substantially in accordance with the procedure set forth in Sato et al., U.S. Patent No. 4,284,818, example #8.

### Example 22

A 100 ml, four-necked round bottom flask was charged with cyclohexane (19.10 g). To this was added methyltrioctylammonium chloride (2.735 g) and anhydrous aluminum chloride (1.803 g). The mixture was cooled to 20°C and allowed to stir for thirty minutes. A mixture of para-cymene (25.13 g), 2,3-dimethyl-1-butene (7.63 g), and tertiary-butyl chloride (9.52 g) was then added to the flask over a period of 3 hours. When the addition was complete, the reaction was quenched with 15 ml of deionized water. The organic phase was then washed with, in order, 5% HCl, 10% Na₂CO₃, and a 50% brine solution. Each aqueous wash was individually extracted with ethyl ether, and the ether layers combined with the organic phase. The organics were then dried over K₂CO₃ and evaporated to yield a crude product (33.78 g) containing 29.22 weight % HMT (50.40 % molar yield of HMT based on the amount of 2,3-dimethyl-1-butene charged). This Example was repeated to yield a crude produce (31.75 g) containing 30.69 weight % HMT (49.76% molar yield of HMT based on the amount of 2,3-dimethyl-1-butene charged).

Example 22 was carried out in the same manner as Example 21, with the exception that a phase transfer agent, methyltrioctylammonium chloride, was added according to the teachings of this invention. This resulted in a 19% improvement in molar yield of HMT compared with Example 21.

### Example 23

This Example is a larger scale version of Example 22 where the addition rate of reagents was doubled to ascertain the ability of the catalyst to handle larger amounts of reactants on a per unit time basis.

A 500 ml, three-necked round bottom flask was charged with cyclohexane (76.9 g), anhydrous aluminum chloride (7.21 g), and methyltrioctylammonium chloride (10.93 g), and stirred for 0.25 hours at 20°C. A mixture of para-cymene (99.47), 2,3-dimethyl-1-butene (30.21 g, 97% purity), and tertiary-butyl chloride (37.67) was prepared and added to the flask over a period of 1.5 hours. Samples were taken and analyzed every 0.25 hours. Immediately upon completion of the addition the reaction was quenched with water (75 ml) and a sample was taken and analyzed. The remaining product was washed with, in order, 5% aqueous hydrochloric acid, 10% aqueous sodium carbonate, and a 50% brine solution. Each aqueous wash was individually extracted with ethyl ether, and the ether layers combined with the organic phase. The organics were then dried over K₂CO₃ and evaporated to give a crude product (134.47 g) containing 29.6 weight % HMT (47.5 % molar yield of HMT based on the amount of 2,3-dimethyl-1-butene charged).

The results of the samplings are shown in Table A.

**Table A**

| Wt % Analysis of Example 23 Samples* | | | | |
|---|---|---|---|---|
| Sample | Time of Sample (hrs) | Para-Cymene | HMT | HMT ö [Para-Cymene + HMT] |
| A | 0.25 | 8.51 | 7.41 | 0.465 |
| B | 0.50 | 14.41 | 11.77 | 0.450 |
| C | 0.75 | 18.48 | 15.52 | 0.456 |
| D | 1.00 | 21.15 | 17.51 | 0.453 |
| E | 1.25 | 23.69 | 18.69 | 0.441 |
| F | 1.50 | 26.10 | 19.88 | 0.432 |

| | | | | |
|---|---|---|---|---|
| *Wt % data normalized based on throughput to allow easier data comparison. | | | | |

### Example 24 (Comparative Example)

This Example is a larger scale version of Example 21 where the addition rate of reagents was doubled to ascertain the ability of the catalyst to handle larger amounts of reactants on a per unit time basis. After quench and work-up, a product (104.91 g) was obtained which contained 23.3 weight % HMT (31.5% molar yield of HMT based on the amount of 2,3-dimethyl-1-butene charged).

The results of the samplings are shown in Table B.

**Table B**

| Wt % Analysis of Example 24 Samples* | | | | |
|---|---|---|---|---|
| Sample | Time of Sample (hrs) | Para-Cymene | HMT | HMT ö [Para-Cymene + HMT] |
| A | 0.25 | 6.22 | 5.87 | 0.486 |
| B | 0.50 | 15.86 | 9.36 | 0.371 |
| C | 0.75 | 21.75 | 10.92 | 0.334 |
| D | 1.00 | 27.74 | 12.17 | 0.305 |
| E | 1.25 | 30.66 | 12.94 | 0.297 |
| F | 1.50 | 32.54 | 13.07 | 0.287 |

| | | | | |
|---|---|---|---|---|
| *Wt % data normalized based on throughput to allow easier data comparison. | | | | |

It is apparent from a comparison of the results of Examples 23 and 24 that without the phase transfer agent, the AlCl₃ catalyst cannot support the increased addition rate. Only in the initial moments of reaction does the comparative Example 24 compare in the rate to that of Example 23 with added phase transfer agent, an example of the process of the invention.

## Claims

1. A process for producing a polyalkyl tetrahydronaphthalene compound comprising contacting a partially substituted benzene compound, wherein said benzene compound is substituted with two or more substituents that do not substantially interfere with a Friedel-Crafts-type alkylation reaction said substituents including at least one secondary alkyl group having only one alpha-hydrogen, and wherein said benzene compound is unsubstituted in at least one position adjacent to said secondary alkyl group, with an olefinic compound of the Formula 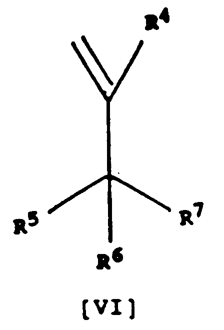 in which R⁴, R⁵, R⁶ and R⁷, independently , are substituents that do not substantially interfere with a Friedel-Crafts-type reaction provided that no more than one of R⁵, R⁶ and R⁷ are H, in the presence of a further reagent and a Lewis acid characterised in that the further reagent is an alkyl halide selected from secondary alkyl halides, tertiary alkyl halides, propargyl halides and allyl halides and in that the process is carried out in the presence of a phase transfer agent.

2. A process according to claim 1 carried out in the substantial absence of elemental iodine.

3. A process according to any preceding claim for producing a polyalkyl tetrahydronaphthalene compound of the formula 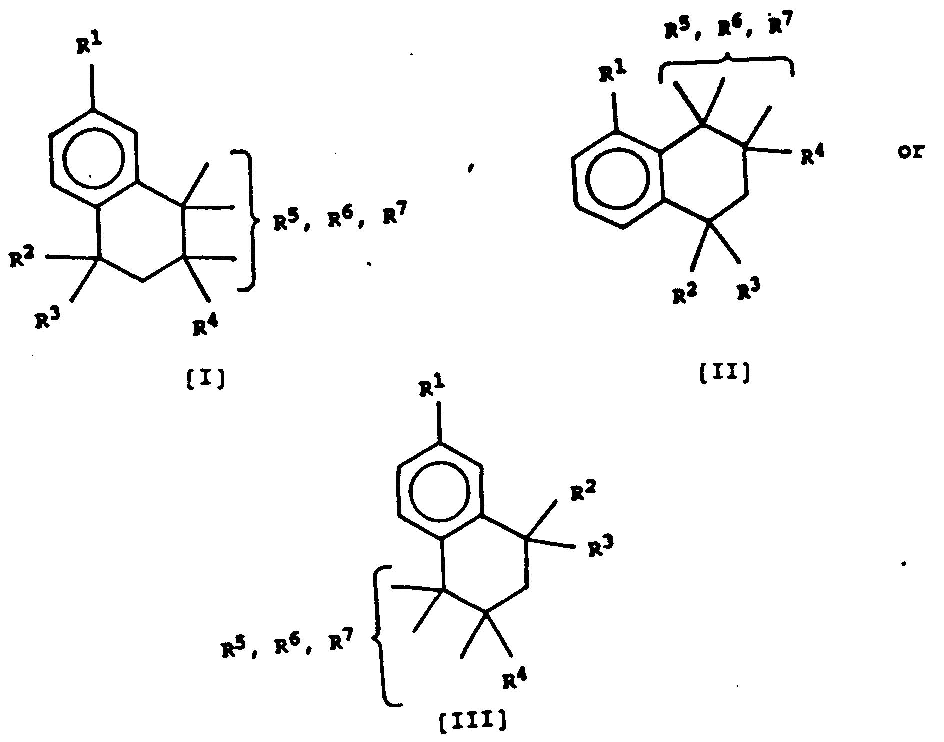 in which the partially substituted benzene compound has the formula 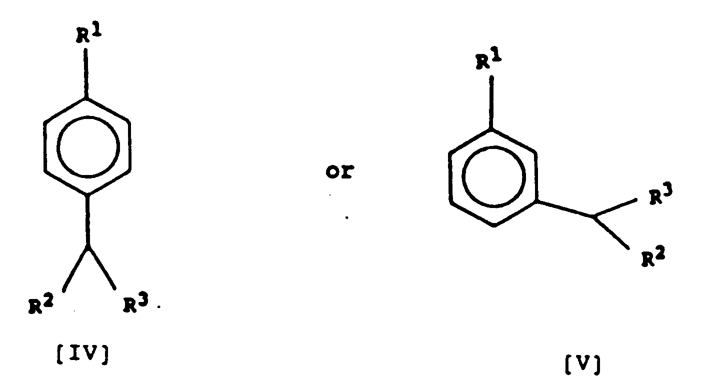 wherein
R¹, R² and R³, independently, are substituents that do not substantially interfere with a Friedel-Crafts-type alkylation reaction, provided that R¹, R² and R³ are each other than H.

4. A process of claim 3 wherein R¹, R² and R³, independently, are a C₁-C₃₀ straight chain, branched or cyclical alkyl; and
R⁴, R⁵, R⁶ and R⁷, independently, are H or a C₁-C₃₀ straight chain, branched or cyclical alkyl and wherein no more than one of R⁵, R⁶ and R⁷ is H.

5. A process of claim 4 wherein said alkyl is a C₁-C₅ straight chain or branched alkyl.

6. A process of claims 4 or 5 wherein said polyalkyl tetrahydronaphthalene compound is of the Formula [I].

7. A process of claim 6 wherein
R¹, R², R³, R⁵, R⁶ and R⁷, independently, are a C₁-C₅ straight chain or branched alkyl; and
R⁴ is H.

8. A process of claim 7 wherein R², R³, R⁵, R⁶ and R⁷ are each methyl and R¹ is selected from methyl, ethyl, n-propyl and t-butyl.

9. A process of claim 6 wherein R¹, R², R³, R⁴, R⁵ and R⁶ are methyl and R⁷ is H.

10. A process of any preceding claim carried out in the presence of an alkyl halide which is a tertiary alkyl halide selected from the group consisting of tertiary-butyl chloride, tertiary-amylchloride, 2-methyl-2-chloropentane, 3-methyl-3-chloropentane, 1,8-dichloro-para-menthane and homologues thereof having fluorine bromine or iodine atoms substituted for the chlorine atom.

11. A process of any preceding claim wherein said Lewis acid is aluminium chloride.

12. A process of any preceding claim carried out in the presence of a phase transfer agent selected from the group consisting of ammonium, phosphonium and sulfonium salts.

13. A process of claim 12 wherein said phase transfer agent is a quaternary ammonium halide.

14. A process of any preceding claim carried out in the presence of a phase transfer agent which is a tertiary amine compound substituted with hydrocarbons.

15. A process of any preceding claim further comprising a solvent.

## Patentansprüche

1. Verfahren zur Herstellung einer Polyalkyltetrahydronaphthalin-Verbindung, umfassend das Kontaktieren einer teilweise substituierten Benzol-Verbindung, worin die Benzol-Verbindung mit zwei oder mehr Substituenten, die eine Alkylierungsreaktion vom Friedel-Crafts-Typ im wesentlichen nicht beeinträchtigen, substituiert ist, wobei die Substituenten mindestens eine sekundäre Alkylgruppe mit nur einem α-Wasserstoff umfassen, und worin die Benzol-Verbindung in mindestens einer zu der sekundären Alkylgruppe benachbarten Stellung unsubstituiert ist, mit einer olefinischen Verbindung der Formel 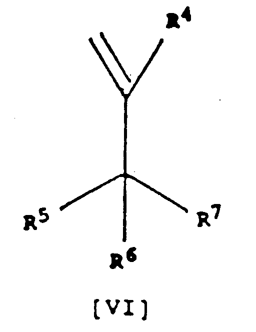 worin R⁴, R⁵, R⁶ und R⁷ unabhängig Substituenten sind, die eine Reaktion vom Friedel-Crafts-Typ im wesentlichen nicht beeinträchtigen, vorausgesetzt, daß nicht mehr als eines von R⁵, R⁶ und R⁷ H ist, in Gegenwart eines weiteren Reagenzes und einer Lewis-Säure, dadurch gekennzeichnet, daß das weitere Reagenz ein aus sekundären Alkylhalogeniden, tertiären Alkylhalogeniden, Propargylhalogeniden und Allylhalogeniden ausgewähltes Alkylhalogenid ist und daß das Verfahren in Gegenwart eines Phasentransfermittels durchgeführt wird.

2. Verfahren nach Anspruch 1, im wesentlichen in Abwesenheit von elementarem Iod durchgeführt.

3. Verfahren nach irgendeinem vorhergehenden Anspruch zur Herstellung einer Polyalkyltetrahydronaphthalin-Verbindung der Formel 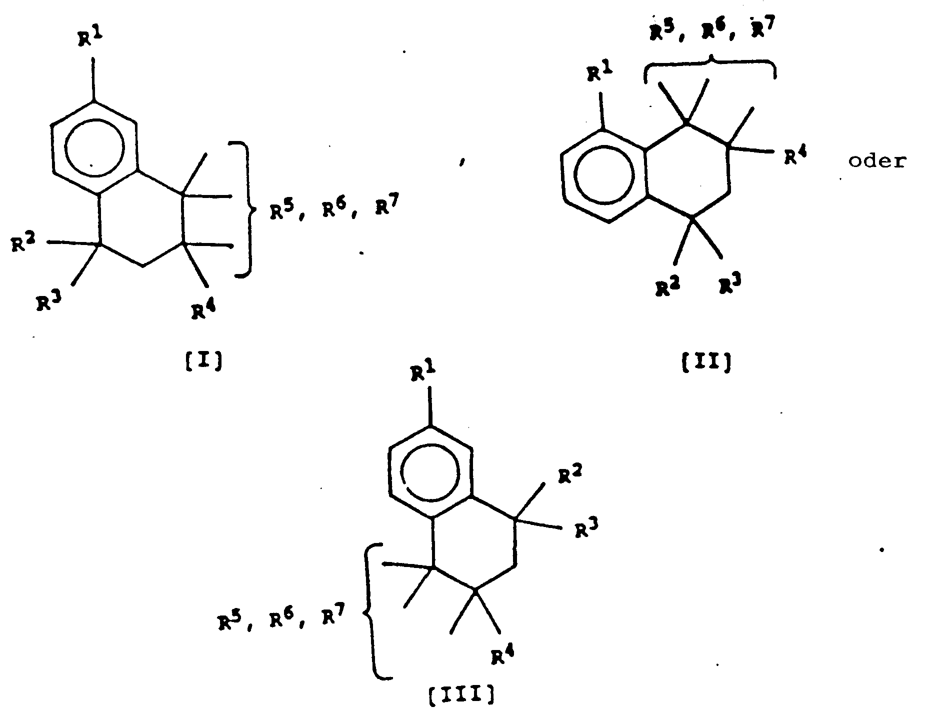 worin die teilweise substituierte Benzol-Verbindung die Formel 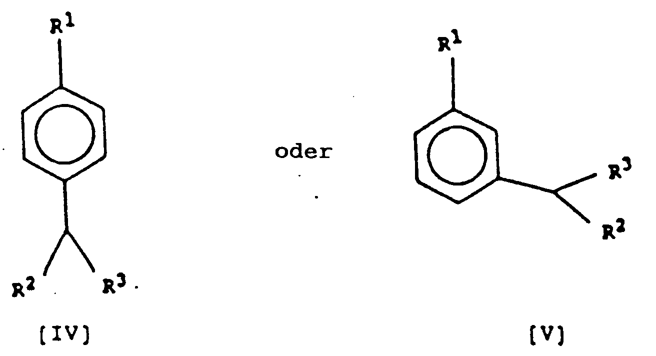 aufweist, worin
R¹, R² und R³ unabhängig Substituenten sind, die eine Alkylierungsreaktion vom Friedel-Crafts-Typ im wesentlichen nicht beeinträchtigen, vorausgesetzt, daß R¹, R² und R³ jeweils von H verschieden sind.

4. Verfahren nach Anspruch 3, worin R¹, R² und R³ unabhängig ein geradkettiges, verzweigtes oder cyclisches C₁-C₃₀-Alkyl sind; und R⁴, R⁵, R⁶ und R⁷ unabhängig H oder ein geradkettiges, verzweigtes oder cyclisches C₁-C₃₀-Alkyl sind und worin nicht mehr als eines von R⁵, R⁶ und R⁷ H ist.

5. Verfahren nach Anspruch 4, worin das Alkyl ein geradkettiges oder verzweigtes C₁-C₅-Alkyl ist.

6. Verfahren nach Anspruch 4 oder 5, worin die Polyalkyltetrahydronaphthalin-Verbindung die Formel (I) aufweist.

7. Verfahren nach Anspruch 6, worin R¹, R², R³, R⁵, R⁶ und R⁷ unabhängig ein geradkettiges oder verzweigtes C₁-C₅-Alkyl sind; und R⁴ H ist.

8. Verfahren nach Anspruch 7, worin R², R³, R⁵, R⁶ und R⁷ jeweils Methyl sind und R¹ aus Methyl, Ethyl, n-Propyl und t-Butyl ausgewählt ist.

9. Verfahren nach Anspruch 6, worin R¹, R², R³, R⁴, R⁵ und R⁶ Methyl sind und R⁷ H ist.

10. Verfahren nach irgendeinem vorhergehenden Anspruch, durchgeführt in Gegenwart eines Alkylhalogenids, bei dem es sich um ein aus der Gruppe bestehend aus tertiärem Butylchlorid, tertiärem Amylchlorid, 2-Methyl-2-chlorpentan, 3-Methyl-3-chlorpentan, 1,8-Dichlor-p-menthan und Homologen davon mit durch Fluor-, Brom- oder Iodatome ersetztem Chloratom ausgewähltes tertiäres Alkylhalogenid handelt.

11. Verfahren nach irgendeinem vorhergehenden Anspruch, worin die Lewis-Säure Aluminiumchlorid ist.

12. Verfahren nach irgendeinem vorhergehenden Anspruch, durchgeführt in Gegenwart eines aus der Gruppe bestehend aus Ammonium-, Phosphonium- und Sulfoniumsalzen ausgewählten Phasentransfermittels.

13. Verfahren nach Anspruch 12, worin das Phasentransfermittel ein quartäres Ammoniumhalogenid ist.

14. Verfahren nach irgendeinem vorhergehenden Anspruch, durchgeführt in Gegenwart eines Phasentransfermittels, bei dem es sich um eine mit Kohlenwasserstoffen substituierte tertiäre Amin-Verbindung handelt.

15. Verfahren nach irgendeinem vorhergehenden Anspruch, welches weiter ein Lösungsmittel umfaßt.

## Revendications

1. Procédé de production d'un composé polyalkyltétrahydronaphtalène comprenant de mettre en contact un composé de benzène partiellement substitué, où ledit composé de benzène est substitué par deux substituants ou plus qui n'interfèrent pas sensiblement avec une réaction d'alkylation de type Friedel-Crafts, lesdits substituants comprenant au moins' un groupe alkyle secondaire ayant seulement un α-hydrogène, et où ledit composé de benzène est non substitué en au moins une position adjacente audit groupe alkyle secondaire, avec un composé oléfinique de formule : 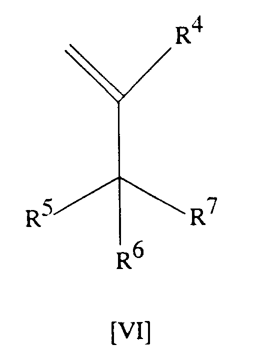 où R⁴, R⁵, R⁶ et R⁷, indépendamment, sont des substituants qui n'interfèrent pas sensiblement avec une réaction de type Friedel-Crafts à condition que pas plus d'un groupe parmi R⁵, R⁶ et R⁷ représente H, en présence de
un autre réactif et un acide de Lewis, caractérisé en ce que l'autre réactif est un halogénure d'alkyle choisi parmi les halogénures d'alkyle secondaire, les halogénures d'alkyle tertiaire, les halogénures de propargyle et les halogénures d'allyle et en ce qu'on réalise le procédé en présence d'un agent de transfert de phase.

2. Procédé selon la revendication 1 réalisé en l'absence substantielle d'iode élémentaire.

3. Procédé selon l'une quelconque des revendications précédentes de production d'un composé polyalkyltétrahydronaphtalène de formule : 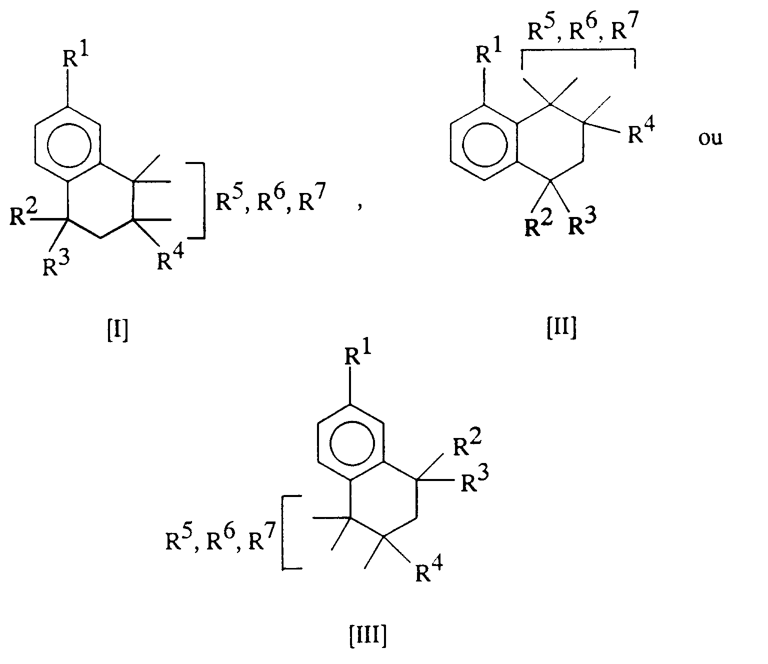 où le composé de benzène partiellement substitué a la formule : 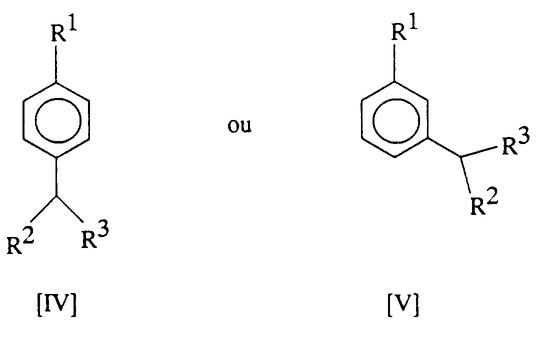 où R¹, R² et R³, indépendamment, sont des substituants qui n'interfèrent pas sensiblement avec une réaction d'alkylation de type Friedel-Crafts, à condition que R¹, R² et R³ soient chacun différents de H.

4. Procédé selon la revendication 3, où R¹, R² et R³, représentent indépendamment un groupe alkyle C₁-C₃₀ à chaîne droite, ramifiée ou cyclique ; et
les groupes R⁴, R⁵, R⁶ et R⁷, représentent indépendamment H ou un groupe alkyle C₁-C₃₀ à chaîne droite, ramifiée ou cyclique et où pas plus d'un groupe parmi R⁵, R⁶ et R⁷ représente H.

5. Procédé selon la revendication 4, où ledit alkyle est un alkyle C₁-C₅ à chaîne droite ou ramifiée.

6. Procédé selon les revendications 4 ou 5, où ledit composé polyalkyltétrahydronaphtalène est un composé de formule [I].

7. Procédé selon la revendication 6, où les groupes R¹, R², R³, R⁵, R⁶ et R⁷ représentent indépendamment un groupe alkyle C₁-C₅ à chaîne droite ou ramifiée ; et
R⁴ est H.

8. Procédé selon la revendication 7, où les groupes R², R³, R⁵, R⁶ et R⁷ représentent chacun un méthyle et R¹ est choisi parmi les méthyle, éthyle, n-propyle et t-butyle.

9. Procédé selon la revendication 6, où les groupes R¹, R², R³, R⁴, R⁵ et R⁶ représentent un méthyle et R⁷ est H.

10. Procédé selon l'une quelconque des revendications précédentes réalisé en présence d'un halogénure d'alkyle qui est un halogénure d'alkyle tertiaire choisi dans le groupe constitué des chlorure de tert-butyle, chlorure de tert-amyle, 2-méthyl-2-chloropentane, 3-méthyl-3-chloropentane, 1,8-dichloro-para-menthane et leurs homologues qui ont des atomes de fluor, de brome ou d'iode à la place de l'atome de chlore.

11. Procédé selon l'une quelconque des revendications précédentes, où ledit acide de Lewis est le chlorure d'aluminium.

12. Procédé selon l'une quelconque des revendications précédentes réalisé en présence d'un agent de transfert de phase choisi dans le groupe constitué des sels d'ammonium, de phosphonium et de sulfonium.

13. Procédé selon la revendication 12, où ledit agent de transfert de phase est un halogénure d'ammonium quaternaire.

14. Procédé selon l'une quelconque des revendications précédentes réalisé en présence d'un agent de transfert de phase qui est un composé amine tertiaire substitué par des hydrocarbures

15. Procédé selon l'une quelconque des revendications précédentes qui comprend en outre un solvant.
